# EUROPEAN PATENT SPECIFICATION

(11) **EP 1 688 111 B1**
(45) Date of publication and mention of the grant of the patent: **03.07.2013**
(21) Application number: 05002584.0
(22) Date of filing: 08.02.2005
(51) Int. Cl.: A61F 13/535, A61F 13/537

(54) **Absorbent article**
Absorbierender Artikel
Article absorbant

(43) Date of publication of application: 09.08.2006
(73) Proprietor: Attends Healthcare AB, 578 24 Aneby (SE)
(72) Inventor: Häkansson, Mikael, 573 37 Tranas (SE); Kvarnström, Irina, 573 39 Tranas (SE); Persson, Christer, 578 31 Aneby (SE); Lindqvist, Arne, 151 35 Södertälje (SE)
(74) Representative: Fritsche, Daniel

(56) References cited:
- EP-A- 0 815 815
- EP-A- 1 097 685
- US-A1- 2003 225 383
- US-A1- 2003 225 384
- US-A1- 2004 186 448

## Description

### Field of the Invention

The invention discloses an absorbent article which comprises, a substantially impervious back sheet, a substantially fluid permeable top sheet,
an absorbent core region between said top sheet and back sheet.

### Technical Background

An absorbent article and in particular an incontinence guard is normally formed of one absorbent body of fluff and often contains super absorbents, which are polymers that are capable of absorbing several times their own weight of water or body liquid.

One difficulty with primarily incontinence guards which are intended to receive and absorb relatively large quantities of liquid, is that the articles often begin to leak before their total absorption capacity has been fully utilized.

From a manufacturing point of view it is also desirable to present incontinence articles which can be utilized effectively since use of unnecessary material leads to a higher cost than necessary. Hence, there is a strive in the business to optimize the capacity of an absorbent article in relation to the materials and compositions used.

Another difficulty encountered with absorbent sanitary articles is keeping the surface of the article which lies against the wearer when in use as dry as possible during the whole of its use period and to prevent so-called rewetting, i.e. to prevent liquid that has already been absorbed being pressed back out of the absorbent article and rewetting the wearer's skin or possibly result in leakage. The rewetting properties of an absorbent article are improved to some extent when the absorbent body comprises super absorbents which bind the absorbed liquid even when the user sits down.

Other aspects of comfort from a wearer's perspective are related to discretion and fitness of the absorbent article. Within the technology of female care lot of efforts have been put into presenting solutions to this problem, which has resulted in small and thin products with suitable capacity for that purpose.

However, urination often results in large quantities of discharge of liquid in short time. Furthermore, it is desirable to prevent leakage of urine and excrements regardless of whether the wearer of the absorbent article is, sitting, moving or laying down. Hence, the high demand on coverage and absorbent capacity, among others, for incontinence articles is making it more difficult to achieve a comfortable and discrete article. The thinner the article the higher the risk of an article which is experienced by the wearer as hard and thus more difficult to form and shape around the pelvic area, which in turn leads to a risk for rubbing against the skin of the wearer.

There exist prior art manufacturing methods which presents absorbent composites of super absorbent material mixed with fluff, suitable for incontinence articles. Due to the relative bulkiness of such absorbent composites they are considered to work relatively well as regards rubbing against the body despite the angularities of their shape.

Consequently, absorbent composites that are thin and have high intake ability are highly desirable. However, there is still a need for an absorbent article, particularly an absorbent article intended for heavy incontinence, which is capable of overcome the issue of uncomfortable rewetting and still being discrete and yet comfortable. It is a further issue to provide an incontinence article which utilizes the material efficiently in order to reduce the necessary amount of material of such articles during manufacturing.

### Summary of the Invention

The object of the present invention is to overcome the above issues and provide an absorbent article, particularly an incontinence guard, according to the preamble of claim 1, wherein there is provided a core region which comprises a first absorbent core and a second absorbent core, said second absorbent core has a longitudinal extent which is 50 to 70% of the longitudinal extent of said first absorbent core. According to the invention the second absorbent core covers 40 to 60% of the face of said first absorbent core, and at least 30% of the face and the length of said second absorbent core extend over the longitudinal mid of said first absorbent core.

Accordingly, 70 to 90%, preferably 75 to 85%, of the total absorption capacity of the absorbent article is within the region of the absorbent core which is covered by the face of the second absorbent core.

Document US 2003/225384 discloses the features of the preamble of claim 1.

The dusting layer creates a barrier which keeps the often sharp and edgy super absorbent particles in place within the mixed sub-layer. Hence, the back sheet and top sheet as well as the skin of the wearer are protected from being scratched. Test result show that a surprisingly low amount of fluff is sufficient in order to create a dusting layer according to the invention.

Accordingly, an absorbent core having a basis weight of fluff in one dusting sub-layer of said absorbent core region below 60 g/m², dry weight basis, is accomplished. Preferably, an absorbent core having a basis weight of fluff in one dusting sub-layer of said absorbent core region below 40 g/m², dry weight basis, is accomplished.

Preferably, said first absorbent core is positioned closer to said back sheet and comprises three sub-layers of which a first mixed sub-layer comprises SAP and fluff and is positioned between two first dusting sub-layers. As mentioned the production method according to the invention enables each absorbent core to be compressed individually before the absorbent article is assembled. Preferably, the first absorbent core has a total dry thickness of 1 to 2 mm, preferably 1.2 to 1.5 mm. Advantageously, said second absorbent core has a total dry thickness of 1.0-3.5 mm, preferably 1.2 to 3.0 mm.

The first and second absorbent cores are compliant and will readily conform to the general shape and contours of the wearer's body.

It is realized that the absorbent article according to the invention is suitable for use as an incontinence guard. According to one preferred embodiment the absorbent article has a front portion and a rear portion provided with a pair of fastening arrangements propagating from said rear portion and adapted to join said front portion from respective side of the waist when said absorbent article is worn by a wearer. A second aspect of the invention is a 2-piece product in which the fastening arrangement according to the above is replaced by stretchable underwear which keeps the absorbent article in place.

Preferably, said second absorbent core comprises two sub-layers of which a second mixed sub-layer comprises super absorbent particles (SAP) and fluff and is positioned sub-adjacent a second dusting sub-layer. In this case the dusting layer of the first absorbent core is used as a common dusting layer for both the cores once they have been assembled.

Alternatively, said second absorbent core comprises three sub-layers of which a second mixed sub-layer comprises super absorbent particles (SAP) and fluff and is positioned between two second dusting sub-layers.

The absorbent core is thin, especially in relation to its capacity, and hence an improved article is accomplished by using a well composed acquisition layer. According to the invention it is provided an acquisition layer immediately below said top sheet for acquisition and distribution of fluid to said absorbent core region, said acquisition layer comprises 60-100%, dry weight basis, twisted stiffened cellulosic fibres. The acquisition layer has a thickness of 2.5 to 3.5 mm.

Advantageously, said first absorbent core has a mid-portion which is narrower than the respective longitudinal end-portions and that said first absorbent core has a smoothly curved shaped contour line along the lateral edges. The basis weight of the total fluff in the first absorbent core is 180-270, preferably 200-250 and more preferably 215-235 gsm.

Still more preferably, said second absorbent core has a mid-portion which is narrower than the respective longitudinal end-portions and that said second absorbent core has a smoothly curved shaped contour line along the lateral edges. The basis weight of the total fluff in the second absorbent core is 200-400, preferably 250-380 and more preferably 280-300 gsm.

### Brief Description of the Drawings

A currently preferred embodiment of the present invention will now be described in more detail, with reference to the accompanying drawings.

Fig. 1 is a plan view of an exploded example of an absorbent article according to an embodiment of the invention.

Fig. 2 is a perspective view of an exploded example of an absorbent article according to an embodiment of the invention.

Fig. 3a is a cross section of one preferred embodiment of the absorbent article.

Fig. 3b is a cross section of one alternative preferred embodiment of the absorbent article.

Fig. 4 is a perspective view of one embodiment of an absorbent article according to the invention in its intended use configuration.

Fig. 5 is a plan view of an exploded example of an absorbent article according to a second aspect of the invention.

### Detailed Description of Preferred Embodiments

A first embodiment of the invention related to an absorbent article will be described in more detail in the following with reference to the accompanying drawings.

### Top sheet and Back sheet

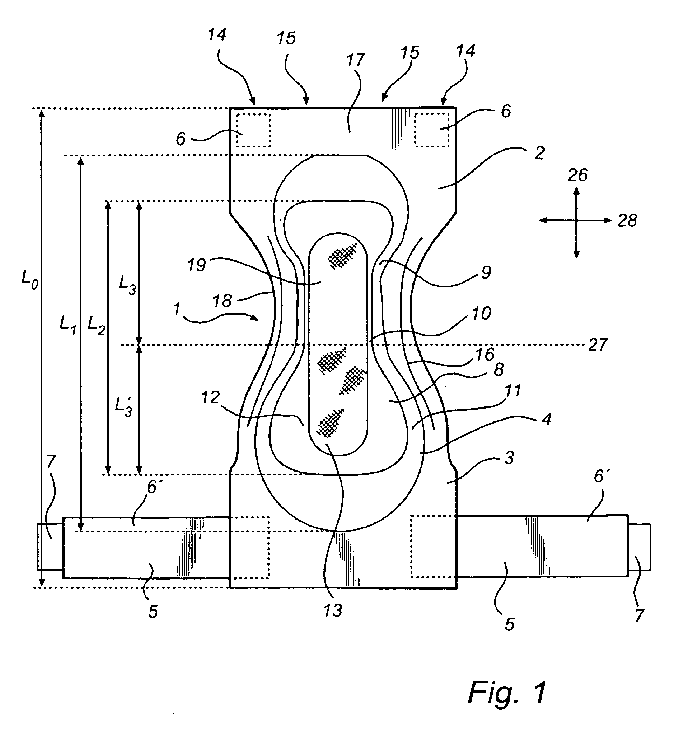

Referring now to Fig. 1, in which a preferred embodiment of the absorbent article 1 is disclosed. The top sheet 19 and the back sheet 18 are co-extensive and have length and width dimensions generally larger than those of the absorbent core. The top sheet 19 is joined with and superimposed on the back sheet 18 thereby forming the periphery of the absorbent article 1. The periphery defines the outer perimeter or the edges of the absorbent article 1.

The top sheet 19 is compliant, soft feeling, and non-irritating to the wearer's skin. Further, the top sheet 19 is liquid pervious permitting liquids to readily penetrate through its thickness. A suitable top sheet 19 can be manufactured from a wide range of materials such as porous foams, reticulated foams, apertured plastic films, natural fibres (e.g., wood or cotton fibres), synthetic fibres (e.g., polyester, polyethen or polypropylene fibres) or from a combination of natural and synthetic fibres. Preferably, the top sheet 19 is made of a combination of hydrophilic and hydrophobic material preferably positioned in different zones over the top sheet 19 in order to meet different demands e.g. isolate the wearer's skin from liquids in the absorbent core.

There are a number of manufacturing techniques which can be used to manufacture the top sheet 19. For example, the top sheet 19 can be woven, non-woven, spun bonded, carded, or the like.

The back sheet 18 is impervious to liquids and is preferably manufactured from a thin plastic film, although other flexible liquid impervious materials may also be used. The back sheet 18 prevents the liquid absorbed and contained in the absorbent core from wetting articles which contact the absorbent article 1. Preferably, the back sheet 18 is polyethylene film having a thickness from about 0.012 mm to about 0.055 centimetres, although other flexible, liquid impervious materials can be used. As used herein, the term "flexible" refers to materials which are compliant and which will readily conform to the general shape and contours of the wearer's body.

Further, the back sheet 18 may be "breathable," permitting vapours to escape from the absorbent core while still preventing exudates from passing through the back sheet 18. It is contemplated that a back sheet that is highly breathable but substantially impervious to liquid may be desirable for certain absorbent articles. The size of the back sheet 18 is dictated by the size of the absorbent core and the absorbent article design selected. In a preferred embodiment, the back sheet 18 has a modified hourglass-shape extending beyond the absorbent core a minimum distance of at least about 1.2 centimetres to at least about 5.0 centimetres around the entire periphery.

The top sheet 19 and the back sheet 18 are joined together in any suitable manner. As used herein, the term "joined" encompasses configurations whereby the top sheet 19 is directly joined to the back sheet 18 by affixing the top sheet 19 directly to the back sheet 18, and configurations whereby the top sheet 19 is indirectly joined to the back sheet 18 by affixing the top sheet 19 to intermediate members which in turn are affixed to the back sheet 18. In a preferred embodiment, the top sheet 19 and the back sheet 18 are affixed directly to each other in the absorbent article periphery by attachment means (not shown) such as an adhesive or any other attachment means as known in the art. For example, a uniform continuous layer of adhesive, a patterned layer of adhesive, or an array of separate lines or spots of adhesive can be used to affix the top sheet 19 to the back sheet 18.

### Elastic members

Referring again to fig 1, elastic members 16 are preferably disposed adjacent the periphery of the absorbent article 1, preferably along each longitudinal edge, so that the elastic members tend to draw and hold the absorbent article 1 against the legs of the wearer. Additionally, elastic members can be disposed adjacent either one or both of the waistband regions of the absorbent article 1 to provide a waistband as well as, or rather than leg elastics.

The elastic members are secured to the absorbent article 1 in an elastically contractible condition so that in a normally unrestrained configuration, the elastic members effectively contract or gather the absorbent article 1. The elastic members can be secured in an elastically contractible condition in at least two ways. For example, the elastic members can be stretched and secured while the absorbent article 1 is in an uncontracted condition. Alternatively, the absorbent article 1 can be contracted, for example, by pleating, and the elastic members secured and connected to the absorbent article 1 while the elastic members are in their un-relaxed or un-stretched condition. The elastic members may extend along a portion of the length of the absorbent article 1. Alternatively, the elastic members can extend the entire length of the absorbent article 1, or any other length suitable to provide an elastically contractible line. The length of the elastic members is dictated by the absorbent article design.

### Acquisition layer

One essential element of the absorbent structures hereof is an upper fluid acquisition/distribution layer which comprises a combination of a hydrophilic fibrous material, described more fully hereinafter. This fluid acquisition/distribution layer serves to quickly collect and temporarily hold discharged body fluid. A portion of discharged fluid may, depending upon the wearer's position, permeate the acquisition/distribution layer and be absorbed by the absorbent region in the area proximate to the discharge. However, since fluid is typically discharged in gushes, the absorbent region in such area may not absorb the fluid as quickly as it is discharged. Therefore, the upper acquisition/distribution layer hereof also facilitates transport of the fluid from the point of initial fluid contact to other parts of the acquisition/distribution layer.

As previously noted, the fluid acquisition/ distribution layer is a web comprising stiffened cellulosic fibres. The acquisition layer comprises from about 60% to 100% of these fibres and from 0% to about 50% of a binding means. Suitable binding means are discussed below. The acquisition layer 13 has a thickness of 2.5 to 3.5 mm.

The fluid distribution function of the acquisition/distribution layer is of particular importance in order to more fully utilize the capacity of the absorbent region. The presence of substantial amounts of super absorbent materials in the acquisition/distribution layer which swell upon contact with fluids is believed to adversely affect this function of the acquisition/distribution layer.

As indicated, the acquisition/distribution layer of the article is preferably elongated. For purposes of this invention, this means that the acquisition/distribution layer is elongated if it is of unequal length and width in the unfolded, flat configuration. The acquisition/distribution layer in the unfolded configuration can be of any desired shape, for example, rectangular, smoothly curved hourglass-shaped, oval, oblong or hourglass-shaped.

The top surface area of the acquisition/distribution layer will preferably range from about 25% to about 90% of the top surface area of the absorbent core face, and also preferably will not extend beyond the edge of the absorbent region at any outer boundary of the secondary core top surface area if this secondary core region is closest to the acquisition/distribution layer. The acquisition/distribution layer will typically have a top surface area less than about 80% of that of the closest absorbent core layer.

Preferably, there is a margin from the edge of the acquisition/distribution layer to the edge of the absorbent core of at least about 0.5 cm, preferably at least about 1.25 cm, in the regions proximate to where fluid is discharged during use.

The fluid acquisition/distribution layer will generally have an average dry density of less than about 0.30 g/cm ³, measured prior to use, and an average density upon wetting to saturation with Synthetic Urine (1.0% NaCl aqueous solution, with distilled water), on a dry weight basis, of less than about 0.20 g/cm³, preferably less than about 0.15 g/cm³. Also, preferably, the average dry density and density upon wetting to saturation are between about 0.02 g/cm³ and 0.20 g/cm ³, more preferably between about 0.02 g/cm³ and about 0. 15 g/cm³. Unless specifically indicated, all basis weights and density values are calculated on a dry basis. Density and basis weight can be substantially uniform although non-uniform density and/or basis weight, and density and/or basis weight gradients, are meant to be encompassed herein. Thus, the acquisition/distribution layer can contain regions of relatively higher or relatively lower density and basis weight, preferably not exceeding the foregoing ranges. Average dry density and average dry density upon wetting to saturation with Synthetic Urine (1.0% NaCl aqueous solution, with distilled water) values are calculated from basis weight of the dry layer and layer caliper.

The acquisition/distribution layer of the absorbent structures herein essentially comprises a web of hydrophilic chemically stiffened cellulosic fibres. These cellulosic fibres are typically wood pulp fibres which have been stiffened with an intra-fibre chemical stiffening agent.

The fluid acquisition/distribution layer is preferably substantially free of super absorbent material. For purposes herein, "substantially free" of super absorbent material means less than about 2.0%, preferably less than about 1.0%, more preferably zero or essentially zero percent super absorbent material.

As discussed above, the articles of the present invention employ chemically stiffened fibres. As used herein, the term "chemically stiffened fibres" means any fibres which have been stiffened by chemical means to increase stiffness of the fibres under both dry and aqueous conditions. Such means include the addition of chemical stiffening agents which, for example, coat and/or impregnate the fibres. Such means also include the stiffening of the fibres by altering the chemical structure of the fibres themselves, e.g., by crosslinking polymer chains.

For exemplary purposes, polymeric stiffening agents which can coat or impregnate cellulosic fibres include: cationic modified starch having nitrogen-containing groups (e.g., amino groups) latex; wet strength resins such as polyamide- epichlorohydrin resin, polyacrylamide resin, urea formaldehyde and melamine formaldehyde resins, and polyethylenimine resins.

The fibres utilized in the structures herein can also be stiffened by means of chemical reaction. For example, cross linking agents can be applied to the fibres which, subsequent to application, are caused to chemically form intra-fibre cross link bonds. These cross link bonds can increase stiffness of the fibres. Whereas the utilization of intrafibre cross link bonds to chemically stiffen the fibres is preferred, it is not meant to exclude other types of reactions for chemical stiffening of the fibres.

In addition to being hydrophilic, these stiffened fibres remain stiff even upon wetting; thus webs made from them do not collapse, as do webs made from conventional unstiffened fibres when wet. This provides improved ability to acquire and distribute fluids in second and subsequent discharges.

In the more preferred stiffened fibres, chemical processing includes intrafibre cross linking with cross linking agents while such fibres are in a relatively dehydrated, defibrated (i.e, individualized), twisted, curled condition. Suitable chemical stiffening agents include monomeric cross linking agents including, but not limited to, C₂- C₈ dialdehydes and C₂-C₈ monoaldehydes having an acid functionality can be employed to form the cross linking solution. These compounds are capable of reacting with at least two hydroxyl groups in a single cellulose chain or on proximately located cellulose chains in a single fibre. Such cross linking agents contemplated for use in preparing the stiffened cellulose fibres include, but are not limited to, glutaraldehyde, glyoxal, formaldehyde, and glyoxylic acid. Other suitable stiffening agents are polycarboxylates, such as citric acid. The effect of cross linking under these conditions is to form fibres which are stiffened and which tend to retain their twisted, curled configuration during use in the absorbent articles herein.

The preferred stiffened cellulose fibres will have an average dry fibre twist count of at least about 2.7, preferably at least about 4.5 twist, nodes per mm. Furthermore, the average wet fibre twist count of these fibres should preferably be at least about 1.8, preferably at least about 3.0, and should also preferably be at least about 0.5 twist nodes per mm less than the average dry fibre twist count. Even more preferably, the average dry fibre twist count should be at least about 5.5 twist nodes per mm, and the average wet fibre twist count should be at least about 4.0 twist nodes per mm and should also be at least 1.0 twist nodes per mm less than its average dry fibre twist count. Most preferably, the average dry fibre twist count should be at least about 6.5 twist nodes per mm, and the average wet fibre twist count should be at least about 5.0 twist nodes per mm and should also be at least 1.0 twist nodes per mm less than the average dry fibre twist count.

In addition to being twisted, the preferred fibres used in the acquisition/distribution layer of the absorbent structure are also curled. Fibre curl may be described as the fractional shortening of the fibre due to kinks, twists, and/or bends in the fibre. For the purposes of this invention, fibre curl is measured in terms of a two dimensional plane. The extent of fibre curling can be quantified by referencing a fibre curl factor. The fibre curl factor, a two dimensional measurement of curl, is determined by viewing the fibre in a two dimensional plane. To determine curl factor, the projected length of the fibre as the longest dimension of a two dimensional rectangle encompassing the fibre, L_{R}, and the actual length of the fibre, L_{A}, are both measured. The fibre curl factor can then be calculated from the following equation: Curl Factor = (L_{A}/L _{R}) - 1.

Preferably the fibres utilized in the layers of the absorbent core herein will have a curl factor of at least about 0.30, and more preferably will have a curl factor of at least about 0.50.

The degree of stiffening, dependent upon the type and amount of stiffening agent (i.e., cross linking agent) used, the degree of dehydration of the fibres during curing of the cross linking agent, and the curing time and conditions, affect the ability of the fibre to take up fluid and the tendency of the fibre to swell.

The fibre stiffness as it relates to resistance to fibre wall swelling. A characteristic of stiffened fibres, particularly the twisted, curled stiffened fibres is their ability to partially untwist and uncurl upon wetting. Thus, when formed into webs of sufficient density, the webs can expand upon wetting to an equilibrium wet density, which, when calculated, on a dry fibre density, is less than the average dry density (prior to wetting). This accounts for the average dry densities of up to about 0.30 g/cm³ described above, in conjunction with lower average densities upon wetting to saturation. The stiffened cellulosic fibres can be provided in web form by various techniques, including air-laying and wet-laying.

A variety of other factors relating to the fluid acquisition/distribution layer of the absorbent structures herein can be of importance in determining the effectiveness of the resulting absorbent articles. These include shape, basis weight, density, permeability, capillarity and wicking ability, the type and structural integrity, and character of the fibrous material utilized.

In use, disposable absorbent articles or other absorbent articles incorporating the fluid absorbent core of the present invention tend to more quickly and efficiently distribute and store liquids and to remain dry due to the high absorbent capacity of the fluid absorbent members. Disposable absorbent articles incorporating the fluid absorbent cores of the present invention can also be thinner and more flexible.

There exist in the art acquisition layers of other compositions, such as Chemical Thermal Mechanical Pulp (CTMP), CTMP or chemical fluff mixed with synthetic fibres and/or SAP and chemical fluff (of same type as within the absorbent core).

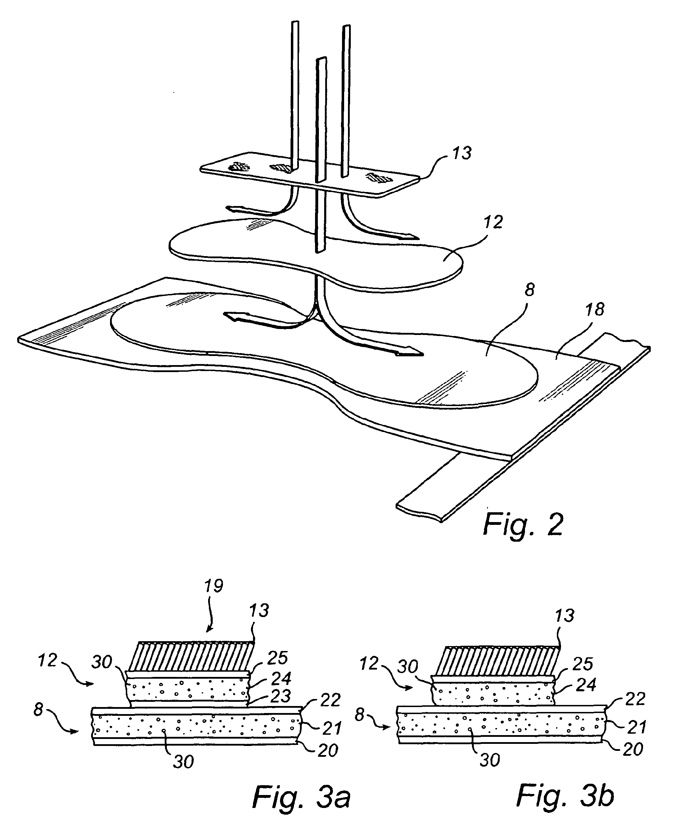

Now, with reference to figs. 1 and 2 the function of the absorbent article 1 and in particular the absorbent core is described in more detail. When used as an absorbent core in a disposable absorbent article 1, a preferred embodiment of the core according to the present invention is positioned such that acquisition/ distribution layer 13 is in fluid communication with top sheet 19, and serves to quickly acquire and partition body exudates from the wearer's body to the generally more absorptive absorbent cores 8, 12. Front portion 2 generally corresponds to the portion of the disposable absorbent article worn in the front of the wearer. Similarly, the rear portion 3 corresponds to the portion of the disposable absorbent article worn in the back of the wearer. The mid zone 10 has a width corresponding to a suitable width for the crotch area of a disposable absorbent article. As well, the length Lₒ of centre section may be varied to provide a suitable fit for various wearer sizes.

### Absorbent core region

The core region comprises a first absorbent core 8 and a second absorbent core 12, said second absorbent core 12 has a longitudinal extent L₂ which is 50 to 70% of the longitudinal extent L₁ of said first absorbent core 8 and covers 40 to 60% of the face of said first absorbent core 8. At least 30% of the face and the length L₃; L₃, of said second absorbent core 12 extend over the longitudinal mid 27 of said first absorbent core 8.

The positioning of the first and second core provides for an efficient distribution and possibly use of the absorbent material of the absorbent article. As will be described in more detail, further on and with reference to figs 3a-3b, 70 to 90%, preferably 75 to 85%, of the total absorption capacity of the absorbent article is within the region of the absorbent core which is covered by the surface of the second absorbent core.

Preferably, said first absorbent core 8 has a mid-portion which is narrower than the respective longitudinal end-portions and that said first absorbent core 8 has a smoothly curved shaped contour line along the lateral edges. Advantageously, said second absorbent core 12 has a mid-portion which is narrower than the respective longitudinal end-portions and that said second absorbent core 12 has a smoothly curved shaped contour line along the lateral edges. The smoothly curved shape together with the parting of the absorbent core into two layers, one first absorbent core and one second absorbent core, and apply said first and second absorbent cores 8, 12 one at the time after individual compression during manufacturing allows for thin layers and yet with high adaptive ability to the shape of the wearer. Said first absorbent core 8 has a total dry thickness of 1 to 2 mm, preferably 1.2 to 1.5 mm. The second absorbent core 12 has a total dry thickness of 1.0-3.5 mm, preferably 1.2 to 3.0 mm.

With reference to fig 3a the absorbent cores according to a first embodiment of the invention is described in more detail. The first absorbent core 8 is positioned closer to said back sheet 18 and comprises three sub-layers 20, 21, 22 of which a first mixed sub-layer 21 comprises SAP and fluff and is positioned between two first dusting sub-layers 20, 22. 8. The basis weight of fluff in one dusting sub-layer 20, 22, 23, 25 of said absorbent core region is below 60 g/m², dry weight basis, preferably below 40 g/m² .The fluff content of said two first dusting sub-layers are 20, 22 is 30 to 40%, dry weight basis, of the total fluff content of said first absorbent core (8).

The second absorbent core 12 comprises three sub-layers 23, 24, 25 of which a second mixed sub-layer 24 comprises super absorbent particles (SAP) and fluff and is positioned between two second dusting sub-layers 23, 25. Super absorbent particles are quite sharp and there is a risk that they damage the back sheet or top sheet if in close contact therewith. By keeping the super absorbent particles in a mixed layer 21, 24 separated from either the top sheet or the back sheet by a dusting layer the absorbent article will be more resistant to leakage due to internal damage. Furthermore, the super absorbent particles will be encapsulated and prevented from falling out and the absorption as well as retention capacity of each absorbent core is improved in that the fluff is able to contribute to absorption in the initial stage until the liquid can be absorbed of the super absorbent particles. The acquisition layer 13 has a thickness of 2.5 to 3.5 mm. 8.

With reference now to fig 3b an alternative embodiment of the second absorbent core is presented in which said second absorbent core 12 comprises two sub-layers 24, 25 of which a second mixed sub-layer 24 comprises super absorbent particles (SAP) and fluff and is positioned sub-adjacent a second dusting sub-layer 25. Apart from that the features of the alternative embodiment are similar to that disclosed by fig 3a and thus not repeated again.

According to a first aspect of the invention fastening arrangements are typically present in the form of a band portion 5 of the absorbent article 1 to provide a fastening system for holding the absorbent article on the wearer. The fastening arrangement 5 depicted are representative only. Preferably, the fastening arrangement is provided with a pair of band portions 5 propagating from a rear portion 3 and adapted to join said front portion 2 from respective side of the waist when said absorbent article 1 is worn by a wearer, wherein said absorbent article 1 is provided with first fastening means 6 and second fastening means 7 for providing said joining. Said first fastening means 6, 6', when joined, provide a retaining effect against skewing of the front portion 2, and each said second fastening means 7, when joined, provides a pull resisting effect in a primarily band portion longitudinal direction propagating along the band portions 5.

Furthermore, said first fastening means 6 are joinable in a respective first attachment zone 14 on the front portion 2 of said absorbent article 1 and said second fastening means 7, 7' are joinable in a respective second attachment zone 15 closer to the line of symmetry of the absorbent article, said respective first and second attachment zone 14, 15 being arranged in a primarily longitudinal direction propagating along the band portions 5.

Preferably, the first fastening means 6, 6' are reconnectable, thus allowing for initial adjustment of the absorbent article 1 before final positioning of said absorbent article 1 to the wearer is accomplished by said second fastening means 7. The first fastening means 6, when joined, provide more pull resistance in a transverse direction in relation to the propagation of the band portions than in a direction along said band portions 5.

The first fastening means 6 is of hook-and-loop type. Preferably, the first fastening means 6 comprises a hook portion which hooks extend substantially downwards in relation to a wearer when said absorbent article is positioned on the body of a wearer. Still more preferably both said first 6, 6' and second fastening means 7 is of hook-and-loop type. More advantageously, said second fastening means 7 comprises a hook portion which hooks are directed away from the symmetry line of the absorbent article 1 when said absorbent article is positioned on the body of a wearer.

Alternatively, at least said second fastening means 7 comprises tape. Preferably, in such embodiment said second fastening means 7 comprises a tape portion which extends substantially longitudinal in relation to the propagation of the band portions 5. The first fastening means 6 could also consist of a tape tab portion which extends substantially transversely in relation to the propagation of the band portions 5.

In one alternative embodiment said front portion comprises a landing zone (not shown) which forms part of any one of said fastening means 6, 6', 7, 7'. The landing zone itself may also be reconnectable although not shown here.

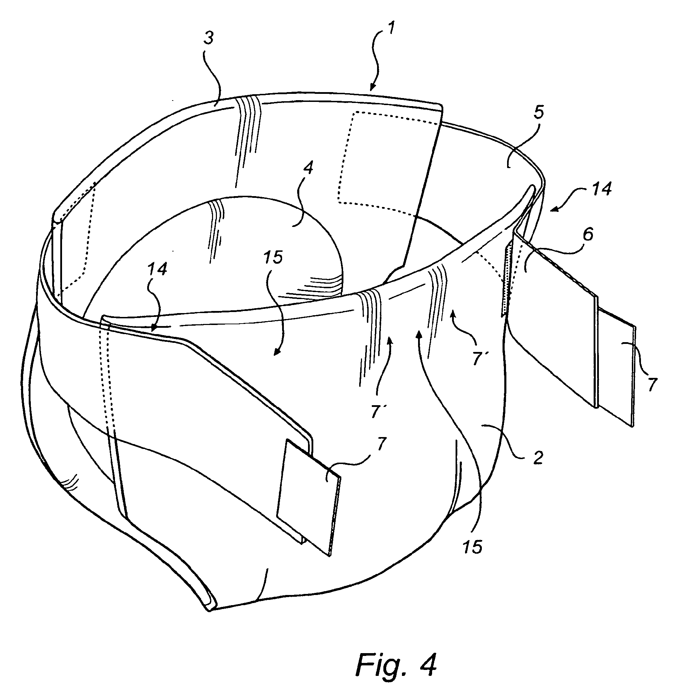

With reference to fig 4 an illustration is provided in which the fastener arrangement is joined by the first fastening means 6 at the corners of the front portion 2 which is denoted as a first attachment zone 14 of the absorbent article 1 and the second fastener means 7 are about to be joined by a second attachment zone 15.

It is realised from the above given examples that there exist various options for the skilled person to design a fastener arrangement that enables the desired functions by using a first and second fastening means and that this could be accomplished by combining the presented features in various ways. Still, even more conventional fastener arrangements may be used in order to provide a comfortable fit. It is realized by the skilled person that the presented options for e.g. back sheet, top sheet, acquisition layer and absorbent core as well as equivalent solutions could be combined in various ways within the scope of protection.

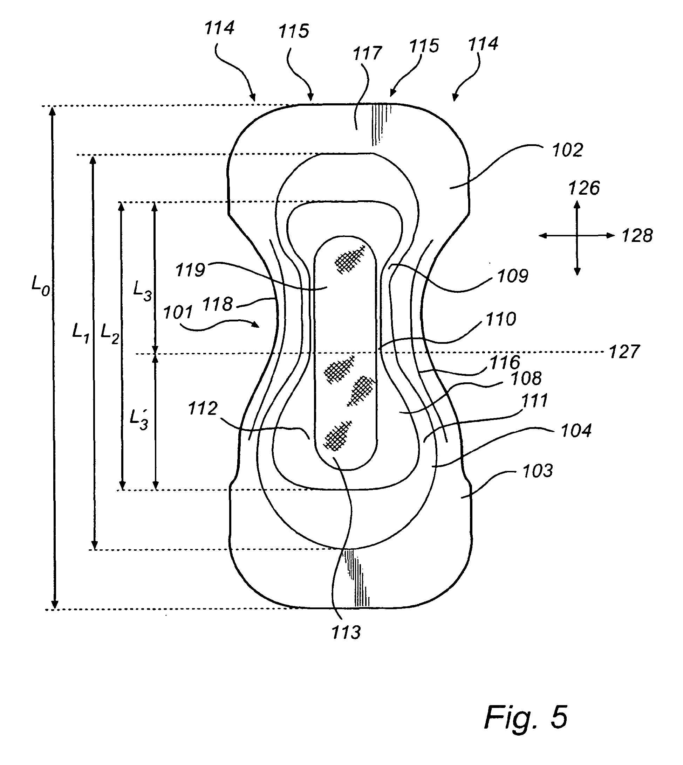

According to a second aspect the absorbent article is disclosed in two-piece form in fig 5. The shape and size as well as composition of materials and layers are not different compared to the first aspect of the invention and thus not repeated again. It is realized by the skilled person that the presented options for e.g. back sheet, top sheet, acquisition layer and absorbent core as well as equivalent solutions could be combined in various ways within the scope of protection. The second aspect of the absorbent article presents no fastener means. Instead an underwear of stretch like material suitable for fitting the absorbent article to the body of a wearer is used. The numbering of the disclosed features of the second aspect in fig. 5 is equivalent to the first aspect although the numbering starts from 100.

In the following table some non-limiting examples of some currently preferred embodiments of absorbent articles are given. It can be seen that the absorption capacity in the target zone is readily improved by adding to the thickness of the secondary core and thus leave room for more fluff and super absorbent particles. The density of super absorbent particles will remain the same.

The overall absorbent capacity can be further improved by adding more super absorbent particles in the mixed first sub-layer 21 and still maintain the thickness of the first absorbent core 8. The capacity is given as the calculated capacity according to the ERT 441.2-02 standard from the Edana organisation. The Edena calculation applies where there is a reference to absorption capacity in this document.

The calculations are based on a super absorbent with a capacity of 33q liquid per q.

| **Layer features:** | | **Alt 1a** | **Alt 1b** | **Alt 2a** | **Alt 2b** |
|---|---|---|---|---|---|
| **Total Core** | | | | | |
| Total core weigth (SAP+Fluff) | g | 61,5 | 81,0 | 81,0 | 100,0 |
| Total Fluff weight | g | 48,0 | 54,0 | 62,0 | 70,0 |
| Total SAP - weight 33XSAP | g | 13,5 | 27,0 | 19,0 | 30,0 |
| Total thickness | mm | 6,3 | 7,0 | 6,3 | 7,0 |
| Theoretical abs.cap. | ml | 638 | 1107 | 875 | 1270 |
| (4xFluff + 33xSAP) | ml | 441 | 627 | 522 | 770 |
| | ml | 539 | 867 | 682 | 996 |
| %Capacity of total in target zone | % | 85% | 78% | 78% | 78% |

| **Acquisition layer** | | | | | |
|---|---|---|---|---|---|
| Thickness, after compression, unpacked product | mm | 3,0 | 3,0 | 3,0 | 3,0 |
| Total amount of Curley fiber | g | 10,5 | 10,5 | 12,3 | 12,3 |

| **First absorbent core** | | | | | |
|---|---|---|---|---|---|
| Thickness, after compression, unpacked product | mm | 1,3 | 1,3 | 1,3 | 1,3 |
| Total core weigth (SAP+Fluff), first absorbent core | g | 31,7 | 40,3 | 45,5 | 50,0 |
| First absorbent core area | m² | 0,130 | 0,130 | 0,176 | 0,176 |
| Fluff, Total Basis weight first absorbent core | g/m² | 225 | 225 | 225 | 225 |
| Each dusting layer, Basis weight first absorbent core | g/m² | 40 | 40 | 40 | 40 |
| SAP-amount first absorbent core | g | 2,4 | 11,0 | 5,9 | 10,4 |

| **Second absorbent core (2 dusting layers)** | | | | | |
|---|---|---|---|---|---|
| Thickness, after compression, unpacked product | mm | 2,0 | 2,7 | 2,0 | 2,7 |
| Total core weight (SAP+Fluff), sec. core | g | 29,8 | 40,7 | 35,5 | 50,0 |
| Total Fluffamount sec. core | g | 18,8 | 24,8 | 22,4 | 30,4 |
| Each dusting layer, Basis weight, sec core | g/m² | 40 | 40 | 40 | 40 |
| SAP-amount sec. core | g | 11,1 | 16,0 | 13,1 | 19,6 |

The super absorbent particle (SAP) concentration, dry weight basis, of said second mixed sub-layer 24 of said second absorbent core 12 is 1.5 to 4 times greater than in said first mixed sub-layer 21.

## Claims

1. An absorbent article (1) extending along a longitudinal axis from its front portion (2) towards its rear portion (3), the article comprising:
an impervious back sheet (18), a fluid permeable top sheet (19),
an absorbent core region between said top sheet (19) and said back sheet (18), and an acquisition layer (13) arranged between said top sheet and said absorbent core region,
said core region comprises a first absorbent core (8) and a second absorbent core (12), the first absorbent core being arranged between the second absorbent core and the back sheet, each first and second core having a longitudinal extent along the longitudinal axis of the article,
said second absorbent core (12) has a longitudinal extent which is 50 to 70% of the longitudinal extent of said first absorbent core (8),
**characterized in that** the second absorbent core (12) covers 40 to 60% of a face of said first absorbent core (8) facing the second absorbent core,
and at least 30% of a face of the second absorbent core facing the first absorbent core and the length of said second absorbent core (12) extend over the longitudinal mid of said first absorbent core (8), wherein 70 to 90%, preferably 75 to 85%, of the total absorption capacity of the absorbent article is within a region of the absorbent core which is covered by the face of the second absorbent core.

2. The absorbent article (1) according to claim 1, wherein said absorbent article is an incontinence guard article.

3. The absorbent article (1) according to any one of claims 1-2, wherein said first absorbent core (8) has a total dry thickness of 1 to 2 mm, preferably 1.2 to 1.5 mm.

4. The absorbent article (1) according to any one of claims 1-3, wherein said second absorbent core (12) has a total dry thickness of 1.0-3.5 mm, preferably 1.2 to 3.0 mm.

5. The absorbent core (1) according to any of claims 1-4, wherein said first absorbent core (8) is positioned closer to said back sheet (18) than said second absorbent core, and comprises three sub-layers (20, 21, 22) of which a first mixed sub-layer (21) comprises super absorbent particles, SAP, and fluff and is positioned between two first dusting sub-layers (20, 22).

6. The absorbent article (1) according to any one of claims 1-5, wherein said second absorbent core (12) comprises two sub-layers (24, 25) of which a second mixed sub-layer (24) comprises super absorbent particles (SAP) and fluff and is positioned sub-adjacent a second dusting sub-layer (25).

7. The absorbent article (1) according to any one of claims 1-6, wherein said second absorbent core (12) comprises three sub-layers (23, 24, 25) of which a second mixed sub-layer (24) comprises super absorbent particles (SAP) and fluff and is positioned between two second dusting sub-layers (23, 25).

8. The absorbent article (1) according to any one of claims 1-7, wherein the basis weight of fluff in one dusting sub-layer (20, 22, 23, 25) of said absorbent core region is below 60 g/m², dry weight basis, preferably below 40 g/m² .

9. The absorbent article (1) according to claim 6 or 7, wherein the super absorbent particle (SAP) concentration, dry weight basis, of said second mixed sub-layer (24) of said second absorbent core (12) is 1.5 to 4 times greater than in said first mixed sub-layer (21).

10. The absorbent article (1) according to any one of claims 1-9, wherein the acquisition layer (13) is positioned immediately below said top sheet (19) for acquisition and distribution of fluid to said absorbent core region, said acquisition layer comprises 60-100%, dry weight basis, twisted stiffened cellulosic fibres.

11. The absorbent article (1) according to claim 10, wherein said acquisition layer (13) has a thickness of 2.5 to 3.5 mm.

12. The absorbent article (1) according to any one of claims 1-11, wherein said first absorbent core (8) has a mid-portion which is narrower than the respective longitudinal end-portions and that said first absorbent core (8) has a smoothly curved shaped contour line along the lateral edges.

13. The absorbent article (1) according to any one of claims 1-12, wherein said second absorbent core (12) has a mid-portion which is narrower than the respective longitudinal end-portions and that said second absorbent core (12) has a smoothly curved shaped contour line along the lateral edges.

## Patentansprüche

1. Absorbierender Artikel (1), der sich entlang einer Längsachse von seinem vorderen Abschnitt (2) zu seinem hinteren Abschnitt (3) erstreckt, wobei der Artikel umfasst:
eine undurchlässige Rückschicht (18), eine fluiddurchlässige Deckschicht (19), eine absorbierende Kernregion zwischen der Deckschicht (19) und der Rückschicht (18), und eine Aufnahmeschicht (13), die zwischen der Deckschicht und der absorbierenden Kernregion angeordnet ist,
wobei die Kernregion einen ersten absorbierenden Kern (8) und einen zweiten absorbierenden Kern (12) umfasst, wobei der erste absorbierende Kern zwischen dem zweiten absorbierenden Kern und der Rückschicht angeordnet ist, wobei sowohl der erste wie auch zweite Kern eine Längenausdehnung entlang der Längsachse des Artikels hat,
wobei der zweite absorbierende Kern (12) eine Längenausdehnung hat, die 50 bis 70% der Längenausdehnung des ersten absorbierenden Kerns (8) ist,
**dadurch gekennzeichnet, dass** der zweite absorbierende Kern (12) 40 bis 60% der Fläche des ersten absorbierenden Kerns (8) bedeckt, die dem zweiten absorbierenden Kern zugewandt ist,
und mindestens 30% einer Fläche des zweiten absorbierenden Kerns, die dem ersten absorbierenden Kern zugewandt ist und die Länge des zweiten absorbierenden Kerns (12) sich über die Mitte in Längsrichtung des ersten absorbierenden Kerns (8) erstrecken, wobei 70 bis 90%, vorzugsweise 75 bis 85%, der gesamten Absorptionskapazität des absorbierenden Artikels in einer Region des absorbierenden Kerns vorhanden sind, die von der Fläche des zweiten absorbierenden Kerns bedeckt ist.

2. Absorbierender Artikel (1) nach Anspruch 1, wobei der absorbierende Artikel ein Inkontinenzschutzartikel ist.

3. Absorbierender Artikel (1) nach einem der Ansprüche 1 bis 2, wobei der erste absorbierende Kern (8) eine Gesamttrockendicke von 1 bis 2 mm, vorzugsweise 1,2 bis 1,5 mm aufweist.

4. Absorbierender Artikel (1) nach einem der Ansprüche 1 bis 3, wobei der zweite absorbierende Kern (12) eine Gesamttrockendicke von 1,0 bis 3,5 mm, vorzugsweise 1,2 bis 3,0 mm aufweist.

5. Absorbierender Artikel (1) nach einem der Ansprüche 1 bis 4, wobei der erste absorbierende Kern (8) näher zur Rückschicht (18) angeordnet ist als der zweite absorbierende Kern und drei Teilschichten (20, 21, 22) umfasst, von welchen eine erste gemischte Teilschicht (21) superabsorbierende Partikel, SAP, und Faserflaum umfasst und zwischen zwei ersten Faserteilschichten (20, 22) positioniert ist.

6. Absorbierender Artikel (1) nach einem der Ansprüche 1 bis 5, wobei der zweite absorbierende Kern (12) zwei Teilschichten (24, 25) umfasst, von welchen eine zweite gemischte Teilschicht (24) superabsorbierende Partikel, SAP, und Faserflaum umfasst und neben einer zweiten Faserteilschicht (25) positioniert ist.

7. Absorbierender Artikel (1) nach einem der Ansprüche 1 bis 6, wobei der zweite absorbierende Kern (12) drei Teilschichten (23, 24, 25) umfasst, von welchen eine zweite gemischte Teilschicht (24) superabsorbierende Partikel, SAP, und Faserflaum umfasst und zwischen zwei zweiten Faserteilschichten (23, 25) positioniert ist.

8. Absorbierender Artikel (1) nach einem der Ansprüche 1 bis 7, wobei das Flächengewicht von Faserflaum in einer Faserteilschicht (20, 22, 23, 25) der absorbierenden Kernregion unter 60 g/m² auf einer Trockengewichtsbasis, vorzugsweise unter 40 g/m² liegt.

9. Absorbierender Artikel (1) nach Anspruch 6 oder 7, wobei die Konzentration der superabsorbierenden Partikel, SAP, auf einer Trockengewichtsbasis der zweiten gemischten Teilschicht (24) des zweiten absorbierenden Kerns (12) 1,5 bis 4 Mal höher als in der ersten gemischten Teilschicht (21) ist.

10. Absorbierender Artikel (1) nach einem der Ansprüche 1 bis 9, wobei die Aufnahmeschicht (13) unmittelbar unter der Deckschicht (19) zum Aufnehmen und Verteilen von Fluid zu der absorbierenden Kernregion positioniert ist, wobei die Aufnahmeschicht 60 bis 100% auf Trockengewichtsbasis verdrillte, versteifte Zellulosefasern enthält.

11. Absorbierender Artikel (1) nach Anspruch 10, wobei die Aufnahmeschicht (13) eine Dicke von 2,5 bis 3,5 mm hat.

12. Absorbierender Artikel (1) nach einem der Ansprüche 1 bis 11, wobei der erste absorbierende Kern (8) einen Mittelabschnitt hat, der schmäler als die entsprechenden Endabschnitte in Längsrichtung ist, und der erste absorbierende Kern (8) eine sanft gekrümmte Konturlinie entlang den seitlichen Rändern hat.

13. Absorbierender Artikel (1) nach einem der Ansprüche 1 bis 12, wobei der zweite absorbierende Kern (12) einen Mittelabschnitt hat, der schmäler als die entsprechenden Endabschnitte in Längsrichtung ist, und der zweite absorbierende Kern (12) eine sanft gekrümmte Konturlinie entlang den seitlichen Rändern hat.

## Revendications

1. Article absorbant (1) s'étendant le long d'un axe longitudinal depuis sa partie frontale (2) vers sa partie arrière (3), cet article comprenant :
un feuillet arrière imperméable (1), un feuillet supérieur perméable au fluide (19), une partie centrale absorbante entre ledit feuillet supérieur (19) et ledit feuillet arrière (18) et une couche de captation (13) disposée entre ledit feuillet supérieur et ladite partie centrale absorbante,
ladite partie centrale comprenant un premier noyau absorbant (8) et un second noyau absorbant (12), le premier noyau absorbant étant disposé entre le second noyau absorbant et le feuillet arrière, chaque premier et second noyau ayant une extension longitudinale le long de l'axe longitudinal de l'article,
ledit second noyau absorbant (12) ayant une extension longitudinale qui représente 50 à 70 % l'extension longitudinale dudit premier noyau absorbant (8),
**caractérisé en ce que** le second noyau absorbant (12) couvre 40 à 60 % d'une face dudit premier noyau absorbant (8) faisant face au second noyau absorbant,
et au moins 30 d'une face du second noyau absorbant fait face au premier noyau absorbant et la longueur dudit second noyau absorbant (12) s'étend sur le centre longitudinal dudit premier noyau absorbant (8), 70 à 90 %, de préférence 75 à 85 %, de la capacité d'absorption totale de l'article absorbant se situant dans une zone du noyau absorbant qui est couverte par la face du second noyau absorbant.

2. Article absorbant (1) selon la revendication 1, ledit article absorbant étant un article de protection pour les incontinents.

3. Article absorbant (1) selon l'une quelconque des revendications 1 et 2, dans lequel ledit premier noyau absorbant (8) a une épaisseur totale à sec de 1 à 2 mm, de préférence 1,2 à 1,5 mm.

4. Article absorbant (1) selon l'une quelconque des revendications 1 à 3, dans lequel ledit second noyau absorbant (12) a une épaisseur totale à sec de 1,0 à 3,5 mm, de préférence 1,2 à 3,0 mm.

5. Article absorbant (1) selon l'une quelconque des revendications 1 à 4, dans lequel ledit premier noyau absorbant (8) est positionné plus près dudit feuillet arrière (18) que ledit second noyau absorbant (12) et comprend trois sous-couches (20, 21, 22) dont une première sous-couche mixte (21) comprend des particules super-absorbantes (SAP) et du duvet de cellulose et est positionnée entre deux premières sous-couches poudreuses (20, 22).

6. Article absorbant (1) selon l'une quelconque des revendications 1 à 5, dans lequel ledit second noyau absorbant (12) comprend deux sous-couches (24, 25) dont une seconde sous-couche mixte (24) comprend des particules super-absorbantes (SAP) et du duvet de cellulose et est positionnée sous-adjacente à une seconde sous-couche poudreuse (25).

7. Article absorbant (1) selon l'une quelconque des revendications 1 à 6, dans lequel ledit second noyau absorbant (12) comprend trois sous-couches (23, 24, 25) dont une seconde sous-couche mixte (24) comprend des particules super-absorbantes (SAP) et du duvet de cellulose et est positionnée entre deux secondes sous-couches poudreuses (23, 25).

8. Article absorbant (1) selon l'une quelconque des revendications 1 à 7, dans lequel le poids de base de duvet de cellulose dans une sous-couche poudreuse (20, 22, 23, 25) de ladite zone centrale absorbante est inférieur à 60 g/m², base de poids à sec, de préférence inférieur à 40 g/m².

9. Article absorbant (1) selon la revendication 6 ou 7, dans lequel la concentration de particules super-absorbante (SAP), base de poids à sec, de ladite seconde sous-couche mixte (24) dudit second noyau absorbant (12) est 1,5 à 4 fois supérieure à celle de ladite première sous-couche mixte (21).

10. Article absorbant (1) selon l'une quelconque des revendications 1 à 9, dans lequel la couche de captation (13) est positionnée juste en-dessous dudit feuillet supérieur (19) pour capter et répartir le fluide vers ladite zone centrale absorbante, ladite couche de captation comprenant 60 à 100 %, base de poids à sec, de fibres cellulosiques raidies et torsadées.

11. Article absorbant (1) selon la revendication 10, dans lequel ladite couche de captation (13) a une épaisseur de 2,5 à 3,5 mm.

12. Article absorbant (1) selon l'une quelconque des revendications 1 à 11, dans lequel ledit premier noyau absorbant (8) a une partie médiane qui est plus étroite que les parties terminales longitudinales respectives et que ledit premier noyau absorbant (8) a une ligne de contour légèrement incurvée le long des bords latéraux.

13. Article absorbant (1) selon l'une quelconque des revendications 1 à 12, dans lequel ledit second noyau absorbant (12) a une partie médiane qui est plus étroite que les sections terminales longitudinales respectives et que ledit second noyau absorbant (12) a une ligne de contour légèrement incurvée le long des bords latéraux.
